# EUROPÄISCHE PATENTSCHRIFT

(11) **EP 3 624 883 B1**
(45) Veröffentlichungstag und Bekanntmachung des Hinweises auf die Patenterteilung: **29.06.2022**
(21) Anmeldenummer: 18725210.1
(22) Anmeldetag: 16.05.2018
(51) Int. Cl.: A61M 16/20, A61M 16/08

(54) **EXSPIRATIONSVENTIL FÜR EINE BEATMUNGSVORRICHTUNG MIT GERÄUSCHMINDERNDEM STRÖMUNGSWIDERSTAND**
EXHALATION VALVE FOR A VENTILATOR APPARATUS WITH NOISE-REDUCING FLOW RESISTANCE
SOUPAPE D'EXPIRATION D'UN DISPOSITIF RESPIRATOIRE AVEC RÉSISTANCE À L'ÉCOULEMENT MINIMISANT LES BRUITS

(30) Priorität: 18.05.2017 DE 102017208421
(43) Veröffentlichungstag der Anmeldung: 25.03.2020
(73) Patentinhaber: Hamilton Medical AG, 7402 Bonaduz (CH)
(72) Erfinder: HUNGER, Jan, 7440 Andeer (CH)
(74) Vertreter: Ruttensperger Lachnit Trossin Gomoll
(86) Internationale Anmeldenummer: PCT/EP2018/062793
(87) Internationale Veröffentlichungsnummer: WO 2018/210956

(56) Entgegenhaltungen:
- EP-A2- 1 197 238
- EP-A2- 1 491 227
- EP-B1- 2 663 354
- WO-A1-02/076544
- CN-A- 101 856 534
- CN-A- 102 274 568
- DE-A1- 19 817 332
- DE-U1- 8 816 546
- US-A1- 2009 044 810
- US-A1- 2010 006 101
- US-A1- 2016 058 969
- US-A1- 2016 256 074

## Beschreibung

Die vorliegende Erfindung betrifft eine Exspirationsventilanordnung für eine Exspirationsleitung einer Beatmungsvorrichtung zur künstlichen Beatmung von Patienten.

Exspirationsventilanordnungen, wie die oben Beschriebene, sei es nun mit oder ohne Strömungswiderstandsformation im stromabwärtigen Atemgaskanal, werden in Beatmungsvorrichtungen zur wenigstens unterstützenden künstlichen Beatmung von Patienten verwendet, um einen den natürlichen Atmungszyklen richtungsmäßig entsprechenden Atemgasfluss zu erzeugen. Die Beatmungsvorrichtungen weisen üblicherweise eine Inspirationsleitung und eine Exspirationsleitung auf mit einer in der Inspirationsleitung vorgesehenen Inspirationsventilanordnung und mit einer in der Exspirationsleitung angeordneten Exspirationsventilanordnung. Die Inspirationsventilanordnung lässt bei makroskopischer Betrachtung der Vorgänge im Wesentlichen nur einen inspiratorischen Atemgasfluss zum Patienten hin zu. Die Exspirationsventilanordnung lässt bei ebenso makroskopischer Betrachtung im Wesentlichen nur einen exspiratorischen Atemgasfluss in Exspirationsströmungsrichtung vom Patienten weg zu.

Bei stärker detaillierter Betrachtung der Exspirationsventilanordnung können dort über das bloße Öffnen und Schließen hinaus weitere für einen Beatmungsvorgang wichtige Vorgänge stattfinden, wie etwa ein Aufrechterhalten einer residualen Durchströmungsöffnung an der Ventilbaugruppe gegen Ende eines Exspirationsvorgangs, um sicherzustellen, dass in der Exspirationsleitung und damit auch in der mit dieser strömungstechnisch kommunizierenden Patientenlunge ein positiver endexspiratorischer Druck (PEEP = "positive end exspiratory pressure") aufrechterhalten wird.

Die eingangs genannten vorbestimmten Atemgasüberdrucke: erster und zweiter Atemgasüberdruck, müssen weder betragsmäßig übereinstimmen, noch auf demselben Atemgas-Druckniveau liegen.

Da der stromabwärtige Atemgaskanal bei den meisten bekannten Exspirationsventilanordnungen zur Außenumgebung als einer Atemgas-Senke der die Exspirationsventilanordnung tragenden Beatmungsvorrichtung hin öffnet, wird dem Atemgas im stromabwärtigen Atemgaskanal üblicherweise von seinem zur Außenumgebung hin öffnenden Längsende her der Umgebungsdruck aufgezwungen. Daher ist der erste Atemgasüberdruck in der Regel ein Überdruck im stromaufwärtigen Atemgaskanal bezüglich des Drucks der Außenumgebung, wie es für einen Exspirationsvorgang charakteristisch ist. Der zweite Atemgasüberdruck ist dann ein Überdruck des Umgebungsdrucks bezüglich eines im stromaufwärtigen Atemgaskanal herrschenden geringeren Drucks, wie es beispielsweise für einen Inspirationsvorgang charakteristisch ist.

Die Aussage, dass die Ventilbaugruppe bei Vorliegen des ersten Atemgasüberdrucks eine Exspirationsströmung in Exspirationsströmungsrichtung vom stromaufwärtigen Atemgaskanal zum stromabwärtigen Atemgaskanal gestattet und bei Vorliegen des zweiten vorbestimmten Atemgasüberdrucks eine Strömung in entgegengesetzte Richtung verhindert, soll nicht ausschließen, dass dann, wenn von dem ersten und dem zweiten vorbestimmten Atemgasüberdruck abweichende Druckverhältnisse vorliegen, Betriebszustände der Ventilbaugruppe vorliegen können, die eingangs der Anmeldung nicht genannt sind. Entscheidend ist nur, dass bei Vorliegen der genannten Atemgasüberdrucke die genannten Betriebszustände der Ventilbaugruppe vorliegen.

Der erste und der zweite vorbestimmte Atemgasüberdruck können jeweils ein Atemgasüberdruck-Wertebereich sein, um unterschiedliche Patienten und Patiententypen sicher beatmen zu können.

Die Begriffe "stromaufwärtig" und "stromabwärtig" beziehen sich an der Exspirationsventilanordnung jeweils auf die konstruktiv eindeutig an dieser erkennbare Exspirationsströmungsrichtung, die bei Vorliegen des vorbestimmten ersten Atemgasüberdrucks durch die Exspirationsventilanordnung hindurch ermöglicht wird.

Eine gattungsgemäße Exspirationsventilanordnung ist aus der EP 2 663 354 B1 bekannt. Eine weitere Exspirationsventilanordnung ist aus der WO 02/076544 A1 bekannt.

Der stromabwärtige Atemgaskanal der aus der WO 02/076544 A1 bekannten Exspirationsventilanordnung weist eine näher bei der Ventilbaugruppe gelegene venturidüsenförmige Kanalgestalt auf, gefolgt von einem Strömungswiderstandsbauteil in Gestalt eines Metallsiebs.

Nur das Metallsieb der bekannten Exspirationsventilanordnung ist eine Strömungswiderstandsformation im Sinne der vorliegenden Anmeldung, da sich nur diese radial innerhalb der den stromabwärtigen Atemgaskanal nach radial außen begrenzenden Kanalwandung befindet. Die venturidüsenförmige Kanalgestalt ist dagegen durch die Kanalwandung selbst gebildet und kann sich nicht radial innerhalb derselben erstrecken.

Das in Exspirationsströmungsrichtung, also längs der zweiten Kanalbahn, dünne Metallsieb, welches überdies vielmaschig ausgestaltet ist, dient in der bekannten Exspirationsventilanordnung als Strömungswiderstand zur Messung des exspiratorischen Atemgasflusses im stromabwärtigen Atemgaskanal auf Grundlage einer Differenzdruckmessung vor und hinter dem Metallsieb.

Aus der CN 101856534 A ist eine Exspirationsventilanordnung mit drei Strömungswegen bekannt, nämlich einer Atemlufteingang, einem Atemluftausgang und einer Auslassöffnung. Der Atemlufteingang und der Atemluftausgang kommunizieren direkt miteinander. Der Atemlufteingang und die Auslassöffnung kommunizieren über ein zwischenangeordnetes Membranventil, welches zur Einstellung sowohl eines exspiratorischen als auch inspiratorischen Atemluftdrucks in einer Kammer steuerbar ist, mit welcher der Atemlufteingang und der Atemluftausgang verbunden sind.

Aus der CN 102274568 A ist ein eine Exspirationsventilanordnung bekannt, umfassend eine Schale, ein Ventilelement und einen Deckel. In einer Luftauslassleitung der bekannten Ventilanordnung ist ein radial nach innen vorspringender Ring angeordnet, welcher lokal den Durchmesser der Luftauslassleitung reduziert und als Druckausgleichselement für die beiderseits des Ventilelements herrschenden Gasdrücke wirkt. Dadurch wird auch die Geräuschbildung an der Ventilanordnung vermindert.

Aus der US 2016/0058969 A1 ist eine Exspirationsventilanordnung bekannt, welche den Druck von exspiratorischem Atemgas regelt. Mit einem steuerbaren Aktuator regelt die bekannte Exspirationsventilanordnung den Druck und den Auslass von exspiratorischem Atemgas an die Umgebung.

Aus der EP 1 491 227 A2 ist eine Exspirationsventilanordnung mit einer auslenkbaren Strömungswiderstandsklappe im Exspirationskanal bekannt.

Nachteilig an der gattungsgemäßen wie auch an anderen Exspirationsventilanordnungen des Standes der Technik ist das Risiko einer im Beatmungsbetrieb unerwünscht hohen Geräuschemission.

Eine erste Geräuschquelle ist der Ringspalt, der bei Öffnung der als Membranventil ausgestalteten Ventilbaugruppe der bekannten Exspirationsventilanordnung entsteht. Eine zweite Geräuschquelle ist das vielmaschige Metallsieb, bei dessen Durchströmung Turbulenzen oder sogar ein Pfeifen entstehen können. Da derartige Geräuschemissionen im Beatmungsbetrieb periodisch bei jedem Exspirationsvorgang auftreten, können sie auf die Dauer für anwesendes Pflegepersonal sowie für den Patienten selbst sehr belastend werden.

Aufgabe der vorliegenden Erfindung ist es daher, eine Exspirationsventilanordnung der eingangs genannten Art derart weiterzubilden, dass das Risiko einer von ihr ausgehenden unerwünschten Geräuschemission verglichen mit dem Stand der Technik verringert ist.

Diese Aufgabe löst die vorliegende Erfindung durch eine Exspirationsventilanordnung mit den Merkmalen des Anspruchs 1. Die Strömungswiderstandsformation, unterteilt den durchströmbaren Querschnitt des stromabwärtigen Atemgaskanals in nicht mehr als fünf baulich voneinander getrennte Teilquerschnitte oder/und vermindert den durchströmbaren Querschnitt des stromabwärtigen Atemgaskanals längs der zweiten Kanalbahn über eine Länge von nicht weniger als 5 mm.

Durch die Ausgestaltung der Strömungswiderstandsformation derart, dass sie den durchströmbaren Querschnitt des stromabwärtigen Atemgaskanals in nicht mehr als fünf baulich voneinander getrennte Teilquerschnitte unterteilt, entstehen bei der Durchströmung der einzelnen Teilquerschnitte weniger Turbulenzen als bei dem bekannten Metallsieb, da die einzelnen Teilquerschnitte aufgrund ihrer größeren Anzahl jeweils eine größere durchströmbare Teilquerschnittsfläche aufweisen. Bevorzugt unterteilt die Strömungswiderstandsformation den durchströmbaren Querschnitt des stromabwärtigen Atemgaskanals in nicht mehr als drei baulich voneinander getrennte Teilquerschnitte. Vorzugsweise unterscheiden sich die durchströmbaren Flächen der einzelnen Teilquerschnitte voneinander um nicht mehr als 20 %, bezogen auf die im Vergleich jeweils kleinere von zwei Teilquerschnittsflächen.

Zusätzlich oder alternativ wird die genannte Aufgabe dadurch gelöst, dass die Strömungswiderstandsformation den durchströmbaren Querschnitt des stromabwärtigen Atemgaskanals längs der zweiten Kanalbahn über eine Länge von nicht weniger als 5 mm vermindert. Bezugszustand für einen Vergleich ist jeweils die gleiche Exspirationsventilanordnung ohne Strömungswiderstandsformation. Dies bedeutet, dass sich im Bezugszustand an dem Ort, an dem sich an der Exspirationsventilanordnung mit erhöhtem Strömungswiderstand die Strömungswiderstandsformation befindet, radial innerhalb der den stromabwärtigen Atemgaskanal nach radial außen begrenzenden Kanalwandung keine den Strömungsquerschnitt des stromabwärtigen Atemgaskanals vermindernde Struktur befindet.

Bevorzugt werden beide oben genannten Maßnahmen gleichzeitig an einer Exspirationsventilanordnung realisiert. Es reicht jedoch zur Erzielung der erfindungsgemäßen Vorteile aus, wenn nur eine der beiden genannten Maßnahmen an der Exspirationsventilanordnung realisiert ist.

Durch die Erstreckung der den Strömungsquerschnitts vermindernden Strömungswiderstandsformation über wenigstens die genannte Mindestlänge längs der zweiten Kanalbahn kann innerhalb des Bereichs mit vermindertem Strömungsquerschnitt eine laminare Atemgasströmung erzielt werden, was aufgrund des so verringerten Turbulenzgrads der Strömung das Risiko einer Geräuschemission vermindert.

Bevorzugt ist der durchströmbare Querschnitt des stromabwärtigen Atemgaskanals durch die Strömungswiderstandsformation über eine kontinuierlich zusammenhängende Strecke vermindert, um einer Turbulenzbildung im stromabwärtigen Atemgaskanal möglichst wirksam entgegenwirken zu können.

Da ein möglichst ungestörtes Führen der exspiratorischen Atemgasströmung entlang von Wänden im stromabwärtigen Atemgaskanal der Exspirationsventilanordnung einer Turbulenzbildung vorteilhaft entgegenwirkt, ist gemäß einer vorteilhaften Weiterbildung der vorliegenden Erfindung zur Vergrößerung der die exspiratorische Atemgasströmung führenden Wandfläche bei gleicher Führungsstrecke längs der zweiten Kanalbahn daran gedacht, dass die Strömungswiderstandsformation wenigstens in einem längs der zweiten Kanalbahn verlaufenden Axialabschnitt des stromabwärtigen Atemgaskanals in Umfangsrichtung um die den stromabwärtigen Atemgaskanal zentral durchsetzend gedachte zweite Kanalbahn herum über einen Bereich von wenigstens 270° unter Bildung eines von Atemgas durchströmbaren Radialspalts mit radialem Abstand von der Kanalwandung des stromabwärtigen Atemgaskanals angeordnet ist.

Durch die Bildung eines Radialspalts kann die exspiratorische Atemgasströmung im stromabwärtigen Atemgaskanal zwischen zwei jeweils verhältnismäßig großflächigen Begrenzungsflächen geführt werden. Bevorzugt sind die den Radialspalt begrenzenden Flächen makroskopisch glatt, also unprofiliert, um Turbulenzquellen zu vermeiden.

Der Radialspalt muss dabei in den 270° um die zweite Kanalbahn herum keine zusammenhängende Öffnung bilden. Es reicht aus, wenn sich die in Umfangsrichtung erstreckenden mehreren Teil-Radialspalte zu einem Gesamtradialspalt mit wenigstens 270° Umfangserstreckung addieren. Dadurch kann beispielsweise über den Radialspalt überbrückende Radialstege hinweg ein Strömungswiderstandskörper in der Radialmitte des stromabwärtigen Atemgaskanals, bevorzugt von der den stromabwärtigen Atemgaskanal zentral durchsetzend gedachten zweiten Kanalbahn durchdrungen, im stromabwärtigen Atemgaskanal befestigt werden.

Wenn, wie nachfolgend anhand bevorzugter Weiterbildungen der erfindungsgemäßen Exspirationsventilanordnung erläutert wird, die Möglichkeit besteht, die Strömungswiderstandsformation axial außerhalb des stromabwärtigen Atemgaskanals an der Exspirationsventilanordnung festzulegen, kann unter noch weiter verminderter Störung der exspiratorischen Atemgasströmung im stromabwärtigen Atemgaskanal der in dem Axialabschnitt zwischen der Strömungswiderstandsformation und der Kanalwandung des stromabwärtigen Atemgaskanals gebildete Radialspalt bevorzugt ein in Umfangsrichtung geschlossen umlaufender Ringkanal sein.

Zur weiteren Beruhigung bzw. Turbulenzminderung der den stromabwärtigen Atemgaskanal durchströmenden exspiratorischen Atemgasströmung kann der Ringspalt, insbesondere in seiner bevorzugten Gestalt als in Umfangsrichtung geschlossen umlaufender Ringkanal, über wenigstens einen Teil des genannten Axialabschnitts eine konstante Gestalt aufweisen. Diese konstante Gestalt kann bevorzugt eine Teil-Kreisringgestalt oder Kreisringgestalt oder eine Polygongestalt sein.

Zusätzlich oder alternativ kann der Radialspalt, wiederum bevorzugt in seiner Ausbildung als in Umfangsrichtung geschlossen umlaufender Ringkanal, über wenigstens einen Teil des Axialabschnitts eine konstante durchströmbare Querschnittsfläche aufweisen, so dass es zu keiner Verdichtung oder Entspannung des längs des Teil des Axialabschnitts strömenden exspiratorischen Atemgases kommt. Bevorzugt ist zur Realisierung der mit der vorliegend genannten Weiterbildung erzielten Vorteile der Radialspalt in der genannten Weise nicht nur über einen Teil des Axialabschnitts, sondern über den gesamten Axialabschnitt mit konstanter Gestalt oder/und mit konstanter durchströmbarer Querschnittsfläche ausgebildet.

Um eine möglichst große Führungslänge der exspiratorischen Atemgasströmung durch die Strömungswiderstandsformation längs des stromabwärtigen Atemgaskanals zu erreichen, kann überdies vorgesehen sein, dass der Axialabschnitt bzw. allgemein die Strömungswiderstandsformation bis zu dem vom stromaufwärtigen Atemgaskanal fernliegenden Längsende des stromabwärtigen Atemgaskanals reicht.

Zur Erzielung einer möglichst langen Führungslänge einerseits sowie eines möglichst wenig turbulenten Atemgas-Freistrahls in eine Atemgas-Senke am stromabwärtigen Ende einer Exspirationsleitung der Beatmungsvorrichtung, welche in der Regel die Umgebungsatmosphäre sein wird, ist es bevorzugt, wenn die Strömungswiderstandsformation den stromabwärtigen Atemgaskanal axial über sein stromabwärtiges Längsende hinaus überragt. Dann fällt nämlich mit dem Vorbeiströmen an dem stromabwärtigen Längsende zunächst nur die radial äußere Begrenzung der exspiratorischen Atemgasströmung weg, während diese radial innen weiterhin entlang der Strömungswiderstandsformation strömen kann. Dabei ist es zur weiteren Geräuschquellenvermeidung vorteilhaft, wenn die Strömungswiderstandsformation in Exspirationsströmungsrichtung nicht abrupt endet, sondern stattdessen ein sich längs der zweiten Kanalbahn verjüngendes stromabwärtiges Ende aufweist.

Beispielsweise kann sich das stromabwärtige Ende der Strömungswiderstandsformation längs der zweiten Kanalbahn in Exspirationsströmungsrichtung konisch oder torpedoförmig verjüngen, also beispielsweise ein rotationssymmetrisches stromabwärtiges Ende aufweisen, welches sich längs der zweiten Kanalbahn konvex gekrümmt verjüngt. Zur möglichst turbulenzfreien Strömung des exspiratorischen Atemgases längs des stromabwärtigen Atemgaskanals kann zusätzlich oder alternativ zur konstanten Größe oder/und Gestalt des Radialspalts vorgesehen sein, dass die Strömungswiderstandsformation in einem mit der Kanalwandung des stromabwärtigen Atemgaskanals gemeinsamen Erstreckungsabschnitt längs der zweiten Kanalbahn eine längs des Erstreckungsabschnitts in Gestalt und Größe konstante Außenkontur aufweist. Beispielsweise kann die Außenkontur eine zylindrische Außenkontur sein oder eine prismatische Außenkontur, wobei die zylindrische Außenkontur wegen der dann in Umfangsrichtung nicht vorhandenen Ecken und Kanten gegenüber einer prismatischen bevorzugt ist.

Ebenso wie die Strömungswiderstandsformation den stromabwärtigen Atemgaskanal axial über sein stromabwärtiges Längsende hinaus überragen kann, kann die Strömungswiderstandsformation den stromabwärtigen Atemgaskanal axial über sein stromaufwärtiges Längsende, also über sein dem stromaufwärtigen Atemgaskanal näher liegendes Längsende hinaus überragen. Beispielsweise kann die Strömungswiderstandsformation den stromabwärtigen Atemgaskanal längs der zweiten Kanalbahn vollständig durchsetzen und in axialer Richtung zu beiden Seiten hin überragen.

Es sei klargestellt, dass ein Atemgaskanal im Sinne der vorliegenden Anmeldung nur dort besteht, wo der Kanal ausgehend von der den Atemgaskanal zentral durchsetzend gedachten Kanalbahn in Umfangsrichtung geschlossen umlaufend durch eine Kanalwandung begrenzt ist.

Wie bei Exspirationsventilanordnungen des Standes der Technik bereits bekannt ist, können die erste und die zweite Kanalbahn einen Winkel miteinander einschließen, wobei dann bevorzugt die Strömungswiderstandsformation von einem den stromaufwärtigen Atemgaskanal begrenzenden Kanalbauteil in den stromabwärtigen Kanal hinein absteht. Bevorzugt ist der Winkel ein rechter Winkel, wobei bevorzugt die beiden verlängert gedachten Kanalbahnen, insbesondere als geradlinige Kanalachsen, einander schneiden. Es soll jedoch auch eine windschiefe Anordnung der Atemgaskanäle nicht ausgeschlossen sein.

Zur Vermeidung von möglicherweise sich lösenden Bauteilen oder Bauteilabschnitten an der Exspirationsventilanordnung, welche durch eine an diese angeschlossene Exspirations-Verbindungsleitung zum Patienten hin gelangen können, ist bevorzugt vorgesehen, dass die Strömungswiderstandsformation einstückig mit dem Kanalbauteil des stromaufwärtigen Atemgaskanals ausgebildet ist. Beispielsweise kann der stromaufwärtige Atemgaskanal in einem Atemgaskanalrohr geführt sein, welches nach radial innen den stromaufwärtigen Atemgaskanal begrenzt und von welchem radial außen die Strömungswiderstandsformation absteht. Dadurch, dass die beiden Kanalbahnen des stromaufwärtigen und des stromabwärtigen Atemgaskanals einen Winkel miteinander einschließen, vorzugsweise den genannten rechten Winkel, kann die Strömungswiderstandsformation konstruktiv sehr einfach von dem Atemgaskanalrohr des stromaufwärtigen Atemgaskanals radial in ein ebenfalls vorhandenes Atemgaskanalrohr des stromabwärtigen Atemgaskanals hineinragen und dieses bevorzugt längs der zweiten Kanalbahn durchsetzen.

Wenngleich die Exspirationsventilanordnung konstruktiv beliebig ausgestaltet sein kann, solange nur ihre strömungsrichtungsleitende Funktion während der künstlichen Beatmung sichergestellt ist, ist die Exspirationsventilanordnung gemäß der vorliegenden Erfindung bevorzugt in an sich bekannter Weise derart ausgestaltet, dass die Exspirationsventilanordnung eine stromabwärts der Ventilbaugruppe gelegene Ringkammer aufweist, welche den stromaufwärtigen Atemgaskanal umgibt und von welcher der stromabwärtige Atemgaskanal ausgeht.

Dies ermöglicht die Ausbildung des Ventilkörpers als ein ein Längsende des stromaufwärtigen Atemgaskanals überspannender Membrankörper. Der Ventilsitz kann dann an dem Längsende des stromaufwärtigen Atemgaskanals ausgebildet sein.

Der Vorteil eines Membrankörpers als der Ventilkörper der Ventilbaugruppe liegt bei der zuvor genannten Bauweise mit dem Ringkanal darin, dass sowohl der stromaufwärtige Atemgaskanal als auch der stromabwärtige Atemgaskanal auf derselben Seite des Membrankörpers gelegen sein können, so dass die von den genannten Atemgaskanälen weg weisende Seite des Membrankörpers für eine Manipulation zugänglich ist. Beispielsweise kann so die von den Atemgaskanälen weg weisende Seite des Membrankörpers mit Kraft beaufschlagbar sein, sei es mechanisch durch einen Aktuator, wie beispielsweise einem Stößel und dergleichen, oder sei es pneumatisch durch ein Druckgefäß, von welchem der Membrankörper eine druckbeaufschlagte Fläche bilden kann.

Somit ist der Membrankörper für eine Steuerung oder/und Regelung des Betriebs der hier diskutierten Exspirationsventilanordnung in vorteilhafter Weise dazu ausgebildet, mit mehreren unterschiedlichen Kraft- oder Druckquellen beaufschlagt zu werden. Zum einen kann der vom Membrankörper überspannte Teil des stromaufwärtigen Atemgaskanals mit dem vom Patienten kommenden Atemgasdruck beaufschlagt sein. Dann kann der im Ringkanal herrschende Druck einen weiteren Flächenbereich des Membrankörpers mit einem Druck beaufschlagen, wobei dieser weitere Flächenbereich auf derselben Seite gelegen ist wie der vom Druck in stromaufwärtigen Atemgaskanal beaufschlagte Membrankörperbereich. Schließlich kann der Membrankörper auf seiner von den Atemgaskanälen weg weisenden Seite mit einer dritten Kraft- oder Druckquelle beaufschlagt sein. Bei geschlossener Ventilbaugruppe wird der Druck in der Ringkammer in der Regel der Umgebungsdruck sein.

Dementsprechend kann die Exspirationsventilanordnung einen Ventilaktuator aufweisen, durch welchen der Membrankörper in eine Schließrichtung zum Ventilsitz hin mit einer Schießkraft beaufschlagbar ist. Dieser Ventilaktuator kann, wie oben bereits angedeutet, ein mechanischer oder/und ein pneumatischer Ventilaktuator sein.

Weiterhin betrifft die vorliegende Erfindung eine Beatmungsvorrichtung zur künstlichen Beatmung von Patienten mit einer Atemgas-Liefervorrichtung, von welcher eine Inspirationsleitung zu einer Patienten-Beatmungsschnittstelle führt, von der wiederum eine Exspirationsleitung zu einer Atemgas-Senke, wie etwa der Umgebungsatmosphäre, führt. In der Exspirationsleitung ist eine Exspirationsventilanordnung vorgesehen, wie sie oben beschrieben und weitergebildet ist, wobei der stromaufwärtige Atemgaskanal durch einen Abschnitt der Exspirationsleitung, etwa einem Exspirationsschlauch, mit der Patienten-Beatmungsschnittstelle zur Übertragung von exspiratorischem Atemgas von der Patienten-Beatmungsschnittstelle verbunden ist.

Wenn vorliegend ausgesagt ist, dass die Strömungswiderstandsformation den durchströmbaren Querschnitt des stromabwärtigen Atemgaskanals am Ort ihrer Anordnung vermindert, so gilt dies für einen erfindungsgemäßen stromabwärtigen Atemgaskanal, dessen von exspiratorischem Atemgas durchströmbare Querschnittsfläche an einem

Betrachtungsort des stromabwärtigen Atemgaskanals auf etwa 20 bis 30 % der durchströmbaren Querschnittsfläche des stromabwärtigen Atemgaskanals derselben Exspirationsventilanordnung ohne die Strömungswiderstandsformation reduziert wird. Bevorzugt erfolgt eine Verringerung der durchströmbaren Querschnittsfläche auf 22 bis 27 % der ohne Strömungswiderstandsformation durchströmbaren Querschnittsfläche.

Beispielsweise kann die durchströmbare Querschnittsfläche des stromaufwärtigen Atemgaskanals ohne Strömungswiderstandsformation im Bereich von 350 bis 450 mm² liegen und kann die durch die Strömungswiderstandsformation reduzierte durchströmbare Querschnittsfläche am selben Betrachtungsort zwischen 80 und 130 mm² betragen.

Durch die Strömungswiderstandsformation steigt bei Ausbildung der Exspirationsventilanordnung mit der oben genannten Ringkammer um den stromaufwärtigen Atemgaskanal bei geöffneter Ventilbaugruppe der Atemgasdruck in der Ringkammer als in einer ansonsten baugleichen Exspirationsventilanordnung ohne Strömungswiderstandsformation, so dass derselbe als Membranventilkörper ausgebildete Ventilkörper in der Exspirationsventilanordnung mit Strömungswiderstandsformation weiter von seinem Ventilsitz abgehoben wird als wenn die Strömungswiderstandsformation nicht vorgesehen wäre. Damit wird der Öffnungsquerschnitt des zwischen Ventilsitz und Ventilkörper gebildeten Spalts erhöht, wodurch an der Stelle der Durchströmung der Ventilbaugruppe weniger Geräusche durch die Atemgasströmung verursacht werden. Außerdem werden durch die Strömungswiderstandsformation im stromabwärtigen Atemgaskanal weniger Geräusche erzeugt. Die hier vorgestellte Exspirationsventilanordnung ist somit insgesamt leiser als eine ansonsten baugleiche Exspirationsventilanordnung des Standes der Technik ohne die beschriebene Strömungswiderstandsformation.

Die vorliegende Erfindung wird nachfolgend anhand der beiliegenden Zeichnungen näher erläutert. Es stellt dar:
- Fig. 1: eine Längsschnittansicht durch eine erfindungsgemäße Ausführungsform einer Exspirationsventilanordnung in einer die geradlinige erste und die geradlinige zweite Kanalbahn enthaltenden Schnittebene während eines Exspirationsvorgangs,
- Fig. 2: die Ansicht von Fig. 1 am Ende des Exspirationsvorgangs,
- Fig. 3: eine Ansicht der Exspirationsventilanordnung der Fig. 1 und 2 bei Betrachtung längs der Schnittebene III-III von Figur 1 und
- Fig. 4: eine perspektivische Ansicht der Exspirationsventilanordnung der Fig. 1 bis 3.

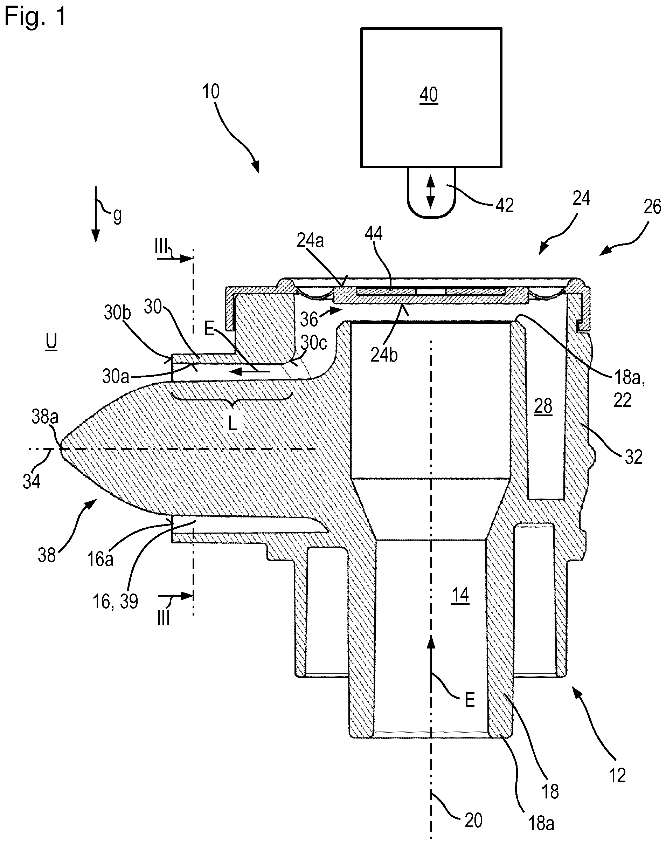

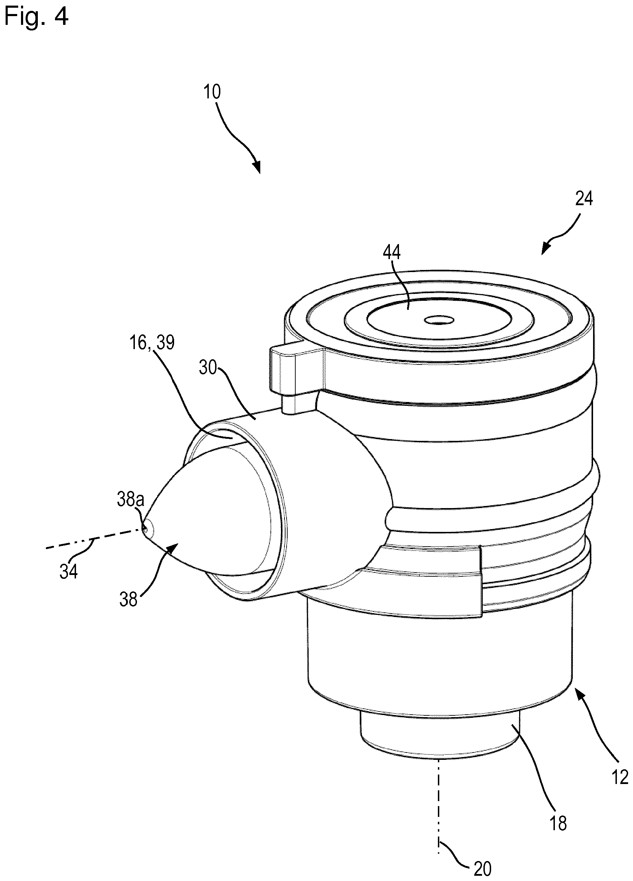

In den Fig. 1 bis 4 ist eine erfindungsgemäße Ausführungsform einer Exspirationsventilanordnung allgemein mit 10 bezeichnet.

Die Exspirationsventilanordnung 10 weist ein einstückig durch Spritzgießen ausgebildetes Leitungsbauteil 12 aus Kunststoff auf, in welchem ein stromaufwärtiger Atemgaskanal 14 und ein stromabwärtiger Atemgaskanal 16 ausgebildet sind.

Der stromaufwärtige Atemgaskanal 14 ist durch ein Atemgasrohr 18 eingefasst und erstreckt sich längs einer geradlinig als erste Kanalachse ausgebildete erste Kanalbahn 20.

Das stromaufwärtige Atemgasrohr 18 und der darin ausgebildete stromaufwärtige Atemgaskanal 14 werden während eines Exspirationsvorgangs mit Atemgas in Exspirationsströmungsrichtung E durchströmt.

An seinem stromaufwärtigen Längsende 18a ist das Atemgasrohr 18 und damit der stromaufwärtige Atemgaskanal 14 an eine in den Figuren nicht dargestellte Exspirationsleitung anschließbar. An seinem entgegengesetzten stromabwärtigen Längsende 18b ist, vorzugsweise einstückig, am stromaufwärtigen Atemgasrohr 18 ein Ventilsitz 22 ausgebildet.

Der Ventilsitz 22 wirkt mit einem Membran-Ventilkörper 24 zusammen und bildet mit diesem eine Ventilbaugruppe 26.

Der Membran-Ventilkörper 24 trennt dann, wenn er auf dem Ventilsitz 22 aufliegt, eine bezüglich der Exspirationsströmungsrichtung E stromabwärts des Ventilsitzes 22 gelegene Ringkammer 28 vom stromaufwärtigen Atemgaskanal 14. Die Ringkammer 28 umgibt einen stromabwärtigen Endabschnitt des stromaufwärtigen Atemgasrohrs 18 radial außen.

Von der Ringkammer 28 ausgehend zweigt der stromabwärtige Atemgaskanal 16 ab und führt wiederum in Exspirationsströmungsrichtung E von der Ringkammer 28 zur Außenumgebung U. Der stromabwärtige Atemgaskanal 16 ist wiederum durch ein Atemgasrohr 30 nach radial außen begrenzt, welches von dem Gehäuse 32 der Ringkammer 28 abzweigt.

Der stromabwärtige Atemgaskanal 16 verläuft längs einer geradlinigen und daher als Kanalachse ausgebildeten zweiten Kanalbahn 34, welche ebenso in der Schnittebene der Darstellung von Fig. 1 gelegen ist, wie die erste Kanalbahn 20. Die Kanalbahnen 20 und 34 sind orthogonal zueinander ausgerichtet und schneiden einander bei verlängert gedachten Kanalbahnen, insbesondere bei verlängert gedachter zweiter Kanalbahn 34.

Die Exspirationsventilanordnung 10 ist in Fig. 1 in ihrer Einbaulage dargestellt, also mit in Schwerkraftwirkungsrichtung g vorbelastetem Membran-Ventilkörper 24. Die erste Kanalbahn 14 verläuft daher im dargestellten Ausführungsbeispiel parallel zur Schwerkraftwirkungsrichtung.

Wegen des in Exspirationsströmungsrichtung E im ersten Atemgaskanal 14 strömenden exspiratorischen Atemgases kommt es zu einem vorbestimmten ersten Druckunterschied zwischen einem im stromaufwärtigen Atemgaskanal 14 herrschenden Druck und dem bei zunächst geschlossener Ventilbaugruppe 26 im stromabwärtigen Atemgaskanal 16 und in der Ringkammer 28 herrschenden Umgebungsdruck, der auch auf der vom Ventilsitz 22 abgewandten Seite 24a des Membran-Ventilkörpers herrscht. Der Membran-Ventilkörper 24 wird daher durch den vorbestimmten ersten Atemgasdruckunterschied vom Ventilsitz 22 abgehoben, so dass zwischen dem Ventilsitz 22 und der dem Ventilsitz zugewandten und in der Schließstellung auf dem Ventilsitz 22 aufliegenden Seite 24b des Membran-Ventilkörpers 24 ein Ventil-Ringspalt 36 gebildet ist.

Eine Exspirationsströmung kann daher die Exspirationsventilanordnung 10 vom stromaufwärtigen Längsende 18a des stromaufwärtigen Atemgasrohrs 18 bis zum stromabwärtigen Längsende 16a des stromabwärtigen Atemgaskanals 16 zur Umgebung U hin durchströmen.

Der stromabwärtige Atemgaskanal 16 ist im dargestellten Beispiel von einer Strömungswiderstandsformation 38 längs der zweiten Kanalbahn 34 vollständig durchsetzt.

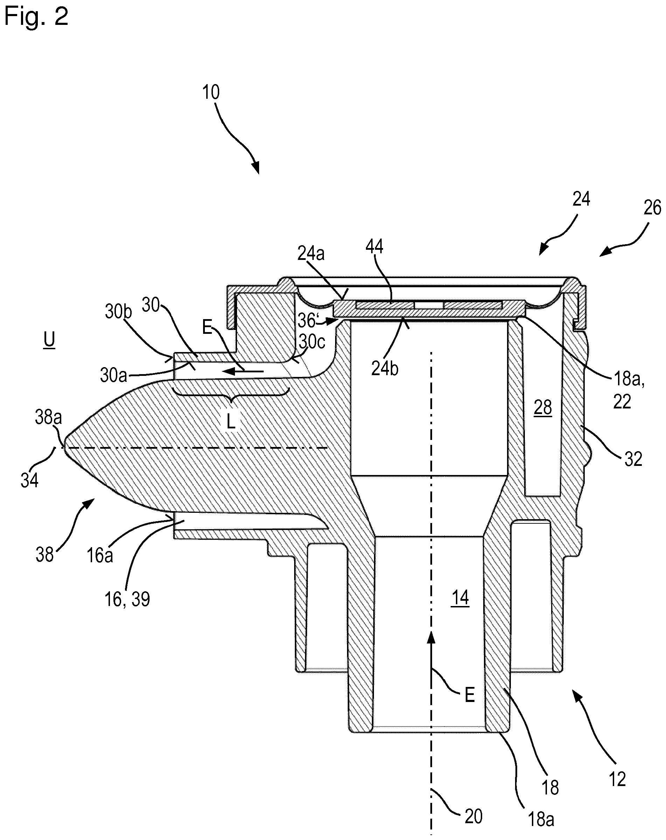

Wie in den Fig. 1 und 2 gut zu erkennen ist, ist die Strömungswiderstandsformation 38 an ihrem stromaufwärtigen Längsende integral mit dem stromaufwärtigen Atemgasrohr 18 ausgebildet und kragt von diesem längs der zweiten Kanalbahn 34, also im vorgestellten Ausführungsbeispiel orthogonal zur ersten Kanalbahn 20, nach radial außen ab.

Die Strömungswiderstandsformation 38 ragt axial bezogen auf die zweite Kanalbahn 34 über das stromabwärtige Längsende 30b des stromabwärtigen Atemgasrohrs 30 und damit über den stromabwärtigen Atemgaskanal 16 hinaus, wo sie sich zu ihrem vom stromaufwärtigen Atemgasrohr 18 fernliegenden Längsende 38a hin verjüngt.

Die Strömungswiderstandsformation 38 ist mit Ausnahme ihrer Anbindung an das stromaufwärtige Atemgasrohr 18 im Wesentlichen rotationssymmetrisch mit der zweiten Kanalbahn 34 als Rotationssymmetrieachse ausgebildet. Der stromabwärtige Atemgaskanal 16 hat daher über seine gesamte Länge L hinweg eine ringkanalförmige Gestalt, wobei der Ringkanal 39 des zweiten Atemgaskanals 16 in Umfangsrichtung um die zweite Kanalbahn 34 unterbrechungsfrei geschlossen umläuft. Die Strömungswiderstandsformation 38 begrenzt den Ringkanal 39 nach radial innen.

Die von der Kanalwandung 30a eingefasste, zur zweiten Kanalbahn 34 orthogonale Querschnittsfläche, welche die durchströmbare Querschnittsfläche des stromabwärtigen Atemgaskanals 16 wäre, wenn die Strömungswiderstandsformation 38 nicht vorhanden wäre, betrüge im dargestellten Beispiel etwa zwischen 410 und 420 mm².

Die das stromabwärtige Atemgasrohr 30 vollständig axial durchsetzende Strömungswiderstandsformation 38 verringert dagegen die tatsächlich durchströmbare kreisringförmige Querschnittsfläche des stromabwärtigen Atemgaskanals 16 auf zwischen 110 und 120 mm², also auf etwas mehr als ein Viertel der ursprünglichen unverminderten Querschnittsfläche.

Hierdurch entstehen zwei Effekte: erstens entsteht durch den erhöhten Strömungswiderstand aufgrund der durch die Strömungswiderstandsformation 38 reduzierten Querschnittsfläche des stromabwärtigen Atemgaskanals 16 im Bereich der Ringkammer 28 bei Durchströmung der Exspirationsventilanordnung 10 mit exspiratorischem Atemgas ein höherer Druck, verglichen mit der ansonsten identischen Exspirationsventilanordnung und identischen exspiratorischen Atemgasströmung, jedoch ohne Strömungswiderstandsformation 38. Dadurch weist der zwischen dem Membran-Ventilkörper 24 und dem Ventilsitz 22 gebildete Ventilspalt 36 eine längs der ersten Kanalbahn 20 zu messende größere Spalthöhe auf als bei ansonsten identischer Situation ohne Strömungswiderstandsformation 38. Dadurch kann der somit flächengrößere Ventilspalt 36 von der exspiratorischen Atemgasströmung mit geringerer bzw. ohne Geräuschentwicklung durchströmt werden.

Zweitens wird die exspiratorische Atemgasströmung durch die sie im stromabwärtigen Atemgasrohr 30 radial innen und radial außen begrenzenden Wände als quasilaminare Gasströmung geführt, so dass sie ebenfalls geräuscharm aus dem stromabwärtigen Atemgasrohr 30 in die Außenumgebung U austritt.

Das sich in Exspirationsströmungsrichtung E verjüngende Längsende 38a der axial über das stromabwärtige Längsende 30b des stromabwärtigen Atemgasrohrs 30 hinausragenden Strömungswiderstandsformation 38 trägt weiter zur Geräuschminderung der erfindungsgemäßen Exspirationsventilanordnung 10 bei.

Der vom stromabwärtigen Atemgasrohr 30 umgebene, sich über die Länge L erstreckende Abschnitt der Strömungswiderstandsformation 38 ist quasi-zylindrisch, d. h. er kann als zylindrisch betrachtet werden und verjüngt sich lediglich geringfügig aufgrund seiner unvermeidlichen Entformungsschräge in Richtung vom stromaufwärtigen Atemgasrohr 18 weg. Der quasi-zylindrische Bereich der Strömungswiderstandsformation 38 im Erstreckungsbereich L des stromabwärtigen Atemgasrohrs 30 von seinem stromaufwärtigen Längsende 30c bis zu seinem stromabwärtigen Längsende 30b hat einen mit der zweiten Kanalbahn 34 eingeschlossenen, für Entformungsschrägen typischen Öffnungswinkel von nicht mehr als 2°, vorzugsweise von nicht mehr als 1,5°. Das sich verjüngende stromabwärtige Längsende 38a der Strömungswiderstandsformation 38, welches in Exspirationsströmungsrichtung E über das stromabwärtige Längsende 30b des stromabwärtigen Atemgasrohrs 30 hinausragt, schließt mit der zweiten Kanalbahn 34 einen wesentlich größeren Konuswinkel ein, welcher sich am stromabwärtigen Längsende der Strömungswiderstandsformation 38 bis zu einem rechten Winkel steigern kann.

Der Membran-Ventilkörper 24 der Exspirationsventilanordnung wird im Betrieb bevorzugt ausschließlich durch die exspiratorische Atemgasströmung angehoben. Er kann jedoch durch einen Aktuator, in Fig. 1 ist beispielhaft ein mechanischer Aktuator 40 dargestellt, in Schließrichtung bewegt werden. Der mechanische Aktuator 40 kann einen Stößel 42 aufweisen, welcher zum Membrankörper 24 hin verlagerbar und von diesem abhebbar ist. Zur Sicherstellung einer möglichst homogenen Einleitung einer vom Aktuator 40 auf den Membran-Ventilkörper 24 ausgeübten Schließkraft ist ein vom Stößel 42 erreichbarer zentraler Bereich des Membran-Ventilkörpers 24 von einer starren Platte 44, insbesondere Metallplatte 44, gebildet.

Vorzugsweise befindet sich in Exspirationsströmungsrichtung E stromabwärts des Ventilsitzes 22 kein Flusssensor und auch kein anderer Sensor, welche die exspiratorische Atemgasströmung in der Nähe ihres Auslasses zur Außenumgebung U hin stört und somit als Geräuschquelle dienen könnten.

In Fig. 2 ist die Exspirationsventilanordnung 10 von Fig. 1 dargestellt, jedoch lediglich mit erheblich verringertem Ventilspalt 36. Zur Unterscheidung ist der geringere Ventilspalt von Fig. 2 mit 36' bezeichnet.

Ein solcher geringer Ventilspalt 36' tritt gegen Ende eines Exspirationsvorgangs auf, um sicherzustellen, dass im stromaufwärtigen Atemgaskanal 12 ein positiver endexspiratorischer Druck (PEEP) herrscht. Er wird durch den Aktuator gezielt eingestellt, um so den PEEP beeinflussen zu können.

Würde ein mit der Exspirationsventilanordnung 10 über eine Exspirationsverbindungsleitung verbundener Patient in der in Fig. 2 gezeigten Betriebssituation der Exspirationsventilanordnung 10 beginnen, einzuatmen, würde ein vorbestimmter zweiter Atemgasüberdruck erzeugt werden, bei welchem stromabwärts des Ventilsitzes 22 etwa der Umgebungsdruck herrscht und im stromaufwärtigen Atemgaskanal 14 ein geringerer Druck als der Umgebungsdruck herrscht. Folglich würde der Membran-Ventilkörper 44 auf den Ventilsitz 22 gedrückt werden und würde eine Strömung von Gas von der Umgebung U entgegen der Exspirationsströmungsrichtung E zum Patienten hin sperren.

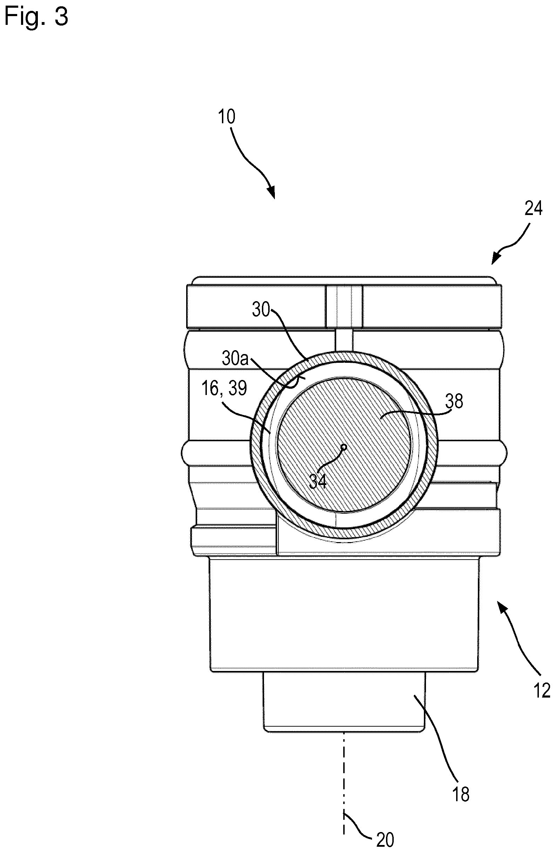

In Fig. 3 ist die kreisringförmige Gestalt des stromabwärtigen Atemgaskanals 16 gut zu erkennen. Wiederum gibt die von der Kanalwandung 30a des stromabwärtigen Atemgasrohrs 30 die Bezugsquerschnittsfläche des Atemgaskanals 16 in einer baugleichen Exspirationsventilanordnung 10 ohne Strömungswiderstandsformation an. hiervon abzuziehen ist im Ausführungsbeispiel die radial innerhalb der Kanalwandung 30a gelegene schraffierte Querschnittsfläche der Strömungswiderstandsformation 38, so dass lediglich der als Ringkanal 39 ausgebildete stromabwärtige Atemgaskanal16 durchströmbar bleibt. Der stromabwärtige Atemgaskanal16 ist nur dort definiert, wo auch das stomabwärtige Atemgasrohr 30 den stromabwärtigen Atemgaskanal16 radial nach außen längs eines geschlossenen Umfangs definiert, also zwischen den Längsenden 30b und 30c des stomabwärtigen Atemgasrohrs 30.

Die Atemgasrohre 18 und 30 sowie die die Ringkammer 28 einfassende Wand 32 sind einstückig ausgebildet, um zu verhindern, dass sich Fügestellen zwischen Bauteilen der Exspirationsventilanordnung 10 lösen.

Die in der vorliegenden Anmeldung vorgestellte Exspirationsventilanordnung 10 ist in Betrieb dauerhaft besonders geräuscharm und schont daher Patient und Pflegekräfte.

## Patentansprüche

1. Exspirationsventilanordnung (10) für eine Exspirationsleitung einer Beatmungsvorrichtung zur künstlichen Beatmung von Patienten, wobei die Exspirationsventilanordnung (10) in einer Exspirationsströmungsrichtung (E) durchströmbar ist und umfasst:
- einen stromaufwärtigen Atemgaskanal (14), welcher sich längs einer ersten Kanalbahn (20) erstreckt und welcher mit einem vom Patienten kommenden Abschnitt der Exspirationsleitung verbunden oder verbindbar ist,
- einen stromabwärtigen Atemgaskanal (16), welcher sich längs einer zweiten Kanalbahn (34) erstreckt und welcher mit einer Atemgas-Senke (U) verbunden oder verbindbar ist, und
- eine einen Ventilkörper (24) und einen Ventilsitz (22) aufweisende Ventilbaugruppe (26), welche zwischen dem stromaufwärtigen und dem stromabwärtigen Atemgaskanal (14, 16) derart vorgesehen ist, dass sie bei einem vorbestimmten ersten Atemgasüberdruck im stromaufwärtigen Atemgaskanal (14) relativ zum stromabwärtigen Atemgaskanal (16) eine exspiratorische Atemgasströmung vom stromaufwärtigen Atemgaskanal (14) in den stromabwärtigen Atemgaskanal (16) zulässt und dass sie bei einem vorbestimmten zweiten Atemgasüberdruck im stromabwärtigen Atemgaskanal (16) relativ zum stromaufwärtigen Atemgaskanal (14) eine Gasströmung vom stromabwärtigen Atemgaskanal (16) in den stromaufwärtigen Atemgaskanal (14) sperrt,
wobei in dem stromabwärtigen Atemgaskanal (16) radial innerhalb der den stromabwärtigen Atemgaskanal (16) nach radial außen begrenzenden Kanalwandung (30a) eine Strömungswiderstandsformation (38) vorgesehen ist, welche den Strömungsquerschnitt des stromabwärtigen Atemgaskanals (16), verglichen mit demselben stromabwärtigen Atemgaskanal (16) ohne Strömungswiderstandsformation (38), am Ort ihrer Anordnung vermindert,
wobei die von exspiratorischem Atemgas durchströmbare Querschnittsfläche des stromabwärtigen Atemgaskanals an einem Betrachtungsort des stromabwärtigen Atemgaskanals auf einen Wert von 20 % bis 30 % der durchströmbaren Querschnittsfläche des stromabwärtigen Atemgaskanals derselben Exspirationsventilanordnung ohne die Strömungswiderstandsformation reduziert ist,
und wobei:
die Strömungswiderstandsformation (38) den durchströmbaren Querschnitt des stromabwärtigen Atemgaskanals (16) in nicht mehr als fünf baulich voneinander getrennte Teilquerschnitte unterteilt
oder/und die Strömungswiderstandsformation (38) den durchströmbaren Querschnitt des stromabwärtigen Atemgaskanals (16) längs der zweiten Kanalbahn (34) über eine Länge von nicht weniger als 5 mm vermindert.

2. Exspirationsventilanordnung (10) nach Anspruch 1,
**dadurch gekennzeichnet, dass** die Strömungswiderstandsformation (38) wenigstens in einem längs der zweiten Kanalbahn (34) verlaufenden Axialabschnitt (L) des stromabwärtigen Atemgaskanals (16) in Umfangsrichtung um die den stromabwärtigen Atemgaskanal (16) zentral durchsetzend gedachte zweite Kanalbahn (34) herum über einen Bereich von wenigstens 270° unter Bildung eines Radialspalts mit radialem Abstand von der Kanalwandung (30a) des stromabwärtigen Atemgaskanals (16) angeordnet ist.

3. Exspirationsventilanordnung (10) nach Anspruch 2,
**dadurch gekennzeichnet, dass** der in dem Axialabschnitt (L) zwischen der Strömungswiderstandsformation (38) und der Kanalwandung (30a) des stromabwärtigen Atemgaskanals (16) gebildete Radialspalt ein in Umfangsrichtung geschlossen umlaufender Ringkanal (39) ist.

4. Exspirationsventilanordnung (10) nach Anspruch 2 oder 3,
**dadurch gekennzeichnet, dass** der Radialspalt, insbesondere als Ringkanal (39), über wenigstens einen Teil des Axialabschnitts (L), vorzugsweise über den gesamten Axialabschnitt (L), eine konstante Gestalt oder/und eine konstante durchströmbare Querschnittsfläche aufweist.

5. Exspirationsventilanordnung (10) nach Anspruch 2 oder 3,
**dadurch gekennzeichnet, dass** der Axialabschnitt (L) bis zu dem stromabwärtigen Längsende (16a) des stromabwärtigen Atemgaskanals (16) reicht.

6. Exspirationsventilanordnung (10) nach einem der vorhergehenden Ansprüche,
**dadurch gekennzeichnet, dass** die Strömungswiderstandsformation (38) den stromabwärtigen Atemgaskanal (16) axial über dessen stromabwärtiges Längsende (16a) hinaus überragt.

7. Exspirationsventilanordnung (10) nach einem der vorhergehenden Ansprüche,
**dadurch gekennzeichnet, dass** die Strömungswiderstandsformation (38) ein sich verjüngendes stromabwärtiges Ende (38a) aufweist.

8. Exspirationsventilanordnung (10) nach einem der vorhergehenden Ansprüche,
**dadurch gekennzeichnet, dass** die Strömungswiderstandsformation (38) in einem mit der Kanalwandung (30a) des stromabwärtigen Atemgaskanals (16) gemeinsamen Erstreckungsabschnitt (L) längs der zweiten Kanalbahn (34) eine längs des Erstreckungsabschnitts (L) in Gestalt und Größe konstante Außenkontur, vorzugsweise zylindrische Außenkontur, aufweist.

9. Exspirationsventilanordnung (10) nach einem der vorhergehenden Ansprüche,
**dadurch gekennzeichnet, dass** die Strömungswiderstandsformation (38) den stromabwärtigen Atemgaskanal (16) axial über dessen stromaufwärtiges Längsende (30c) überragt.

10. Exspirationsventilanordnung (10) nach Anspruch 9,
**dadurch gekennzeichnet, dass** die erste und die zweite Kanalbahn (20, 34) einen Winkel, vorzugsweise einen rechten Winkel, miteinander einschließen, wobei die Strömungswiderstandsformation (38) von einem den stromaufwärtigen Atemgaskanal (14) begrenzenden Kanalbauteil (18) in den stromabwärtigen Atemgaskanal (16) hinein absteht.

11. Exspirationsventilanordnung (10) nach Anspruch 10,
**dadurch gekennzeichnet, dass** die Strömungswiderstandsformation (38) einstückig mit dem Kanalbauteil (18) des stromaufwärtigen Atemgaskanals (14) ausgebildet ist.

12. Exspirationsventilanordnung (10) nach einem der vorhergehenden Ansprüche,
**dadurch gekennzeichnet, dass** sie eine stromabwärts der Ventilbaugruppe (26) gelegene Ringkammer (28) aufweist, welche den stromaufwärtigen Atemgaskanal (14) umgibt und von welcher der stromabwärtige Atemgaskanal (16) ausgeht, wobei die Ventilbaugruppe (16) zwischen dem stromaufwärtigen Atemgaskanal (14) und der Ringkammer (28) angeordnet ist.

13. Exspirationsventilanordnung (10) nach Anspruch 12,
**dadurch gekennzeichnet, dass** der Ventilkörper (24) ein ein Längsende (18a) des stromaufwärtigen Atemgaskanals (14) überspannender Membrankörper (24) ist und dass der Ventilsitz (22) an dem Längsende (18a) des stromaufwärtigen Atemgaskanals (14) ausgebildet ist.

14. Exspirationsventilanordnung (10) nach Anspruch 13,
**dadurch gekennzeichnet, dass** sie einen Ventilaktuator (40) aufweist, durch welchen der Membrankörper (24) in eine Schließrichtung zum Ventilsitz (22) hin mit einer Schließkraft beaufschlagbar ist.

15. Beatmungsvorrichtung zur künstlichen Beatmung von Patienten mit einer Atemgas-Liefervorrichtung, von welcher eine Inspirationsleitung zu einer Patienten-Beatmungsschnittstelle führt, von welcher wiederum eine Exspirationsleitung zu einer Atemgas-Senke (U), wie etwa der Umgebungsatmosphäre (U), führt,
**dadurch gekennzeichnet, dass** in der Exspirationsleitung eine Exspirationsventilanordnung (10) nach einem der vorhergehenden Ansprüche vorgesehen ist, wobei der stromaufwärtige Atemgaskanal durch einen Abschnitt der Exspirationsleitung mit der Patienten-Beatmungsschnittstelle zur Übertragung von exspiratorischem Atemgas von der Patienten-Beatmungsschnittstelle verbunden ist.

## Claims

1. An exhalation valve arrangement (10) for an exhalation line of a ventilation apparatus for artificial ventilation of patients, the exhalation valve arrangement (10) being flowthrough-capable in an exhalation flow direction (E) and encompassing:
- an upstream respiratory gas conduit (14) that extends along a first conduit path (20) and is connected or connectable to a portion, coming from the patient, of the exhalation line;
- a downstream respiratory gas conduit (16) that extends along a second conduit path (34) and is connected or connectable to a respiratory gas sink (U);
- a valve subassembly (26) which comprises a valve body (24) and a valve seat (22) and which is provided between the upstream and the downstream respiratory gas conduit (14, 16) in such a way that, in the context of a predetermined first respiratory gas overpressure in the upstream respiratory gas conduit (14) relative to the downstream respiratory gas conduit (16), it permits an exhalatory respiratory gas flow from the upstream respiratory gas conduit (14) into the downstream respiratory gas conduit (16); and in the context of a predetermined second respiratory gas overpressure in the downstream respiratory gas conduit (16) relative to the upstream respiratory gas conduit (14), it blocks a gas flow from the downstream respiratory gas conduit (16) into the upstream respiratory gas conduit (14),
there being provided in the downstream respiratory gas conduit (16), radially inside the conduit wall (30a) that radially externally delimits the downstream respiratory gas conduit (16), a flow resistance configuration (38) that, at the location where it is arranged, decreases the flow cross section of the downstream respiratory gas conduit (16) compared with the same downstream respiratory gas conduit (16) without a flow resistance configuration (38),
wherein the cross sectional area of the downstream respiratory gas conduit, through which exhalatory respiratory gas can flow, at a location under consideration of the downstream respiratory gas conduit is reduced to a value of 20 % to 30 % of the flowthrough-capable cross sectional area of the downstream respiratory gas conduit of the same exhalation valve arrangement without the flow resistance configuration,
wherein the flow resistance configuration (38) divides the flowthrough-capable cross section of the downstream respiratory gas conduit (16) into no more than five partial cross sections physically separated from one another; and/or the flow resistance configuration (38) decreases the flowthrough-capable cross section of the downstream respiratory gas conduit (16) along the second conduit path (34) over a length of not less than 5 mm.

2. The exhalation valve arrangement (10) according to Claim 1,
**characterized in that** the flow resistance configuration (38) is arranged at a radial distance from the conduit wall (30a) of the downstream respiratory gas conduit (16), at least in an axial portion (L) of the downstream respiratory gas conduit (16) which proceeds along the second conduit path (34), over a region of at least 270° in a circumferential direction around the notional second conduit path (34) passing centrally through the downstream respiratory gas conduit (16), forming a radial gap.

3. The exhalation valve arrangement (10) according to Claim 2,
**characterized in that** the radial gap constituted in the axial portion (L) between the flow resistance configuration (38) and the conduit wall (30a) of the downstream respiratory gas conduit (16) is an annular conduit (39) proceeding continuously in a circumferential direction.

4. The exhalation valve arrangement (10) according to Claim 2 or 3,
**characterized in that** the annular gap, especially constituting an annular conduit (39), has a constant shape and/or a constant flowthrough-capable cross-section over at least part of the axial portion (L), preferably over the entire axial portion (L).

5. The exhalation valve arrangement (10) according to Claim 2 or 3,
**characterized in that** the axial portion (L) extends as far as the downstream longitudinal end (16a) of the downstream respiratory gas conduit (16).

6. The exhalation valve arrangement (10) according to one of the preceding claims,
**characterized in that** the flow resistance configuration (38) protrudes axially beyond the downstream respiratory gas conduit (16) beyond its downstream longitudinal end (16a).

7. The exhalation valve arrangement (10) according to one of the preceding claims,
**characterized in that** the flow resistance configuration (38) has a tapering downstream end (38a).

8. The exhalation valve arrangement (10) according to one of the preceding claims,
**characterized in that** the flow resistance configuration (38) has, in a portion of extent (L) along the second conduit path (34) which is shared with the conduit wall (30a) of the downstream respiratory gas conduit (36), an outer contour that is constant in shape and size along the portion of extent (L), preferably a cylindrical outer contour.

9. The exhalation valve arrangement (10) according to one of the preceding claims,
**characterized in that** the flow resistance configuration (38) protrudes axially beyond the downstream respiratory gas conduit (16) beyond its upstream longitudinal end (30c).

10. The exhalation valve arrangement (10) according to Claim 9,
**characterized in that** the first and the second conduit path (20, 34) enclose an angle, preferably a right angle, between them, the flow resistance configuration (38) of a conduit component (18) which delimits the upstream respiratory gas conduit (14) protruding into the downstream respiratory gas conduit (16).

11. The exhalation valve arrangement (10) according to Claim 10,
**characterized in that** the flow resistance configuration (38) is embodied in one piece with the conduit component (18) of the upstream respiratory gas conduit (14).

12. The exhalation valve arrangement (10) according to one of the preceding claims,
**characterized in that** it comprises an annular chamber (28) which is located downstream from the valve subassembly (26), which surrounds the upstream respiratory gas conduit (14), and from which the downstream respiratory gas conduit (16) proceeds, the valve subassembly (16) being arranged between the upstream respiratory gas conduit (14) and the annular chamber (28).

13. The exhalation valve arrangement (10) according to Claim 12,
**characterized in that** the valve body (24) is embodied as a membrane body (24) that spans a longitudinal end (18a) of the upstream respiratory gas conduit (14); and the valve seat (22) is embodied at the longitudinal end (18a) of the upstream respiratory gas conduit (14).

14. The exhalation valve arrangement (10) according to Claim 13,
**characterized in that** it comprises a valve actuator (40) by which the membrane body (24) can be impinged upon with a closing force in a closing direction toward the valve seat (22).

15. A ventilation apparatus for artificial ventilation of patients, having a respiratory gas supply apparatus from which an inhalation line leads to a patient ventilation interface from which an exhalation line leads in turn to a respiratory gas sink (U), for example the ambient atmosphere (U),
**characterized in that** an exhalation valve arrangement (10) according to one of the preceding claims is provided in the exhalation line, the upstream respiratory gas conduit being connected by way of a portion of the exhalation line to the patient ventilation interface in order to transfer exhalatory respiratory gas from the patient ventilation interface.

## Revendications

1. Agencement de soupape expiratoire (10) pour une ligne d'expiration d'un dispositif de ventilation pour la ventilation artificielle de patients, dans lequel l'agencement de soupape expiratoire (10) peut être traversé dans une direction d'écoulement d'expiration (E) et comprend :
- un canal de gaz respiratoire amont (14) qui s'étend le long d'une première voie de canal (20) et qui est relié ou peut être relié à une partie de la ligne d'expiration venant du patient,
- un canal de gaz respiratoire aval (16) qui s'étend le long d'une deuxième voie de canal (34) et qui est relié ou peut être relié à un puits de gaz respiratoire (U), et
- un agencement de soupape (26) comprenant un corps de soupape (24) et un siège de soupape (22), qui est prévu entre le canal de gaz respiratoire amont et le canal de gaz respiratoire aval (14, 16) de telle sorte, qu'il permet, pour une première surpression de gaz respiratoire prédéterminée dans le canal de gaz respiratoire amont (14) par rapport au canal de gaz respiratoire aval (16), un écoulement de gaz respiratoire expiratoire du canal de gaz respiratoire amont (14) dans le canal de gaz respiratoire aval (16) et qu'il permet, en présence d'une deuxième surpression de gaz respiratoire prédéterminée dans le canal de gaz respiratoire aval (16) par rapport au canal de gaz respiratoire amont (14), un écoulement de gaz du canal de gaz respiratoire aval (16) dans le canal de gaz respiratoire amont (14),
dans lequel une formation de résistance à l'écoulement (38) est prévue dans le canal de gaz respiratoire aval (16) radialement à l'intérieur de la paroi de canal (30a) délimitant le canal de gaz respiratoire aval (16) radialement vers l'extérieur, laquelle formation réduit la section transversale d'écoulement du canal de gaz respiratoire aval (16), comparé avec le même canal de gaz respiratoire aval (16) sans formation de résistance à l'écoulement (38), à l'endroit de sa disposition,
dans lequel la surface de section transversale du canal de gaz respiratoire aval pouvant être traversée par le gaz respiratoire expiré est réduite, à un lieu d'observation du canal de gaz respiratoire aval, à une valeur de 20 % à 30 % de la surface de section transversale pouvant être traversée du canal de gaz respiratoire aval du même agencement de soupape expiratoire sans la formation de résistance à l'écoulement,
et dans lequel :
la formation de résistance à l'écoulement (38) divise la section transversale pouvant être traversée du canal de gaz respiratoire aval (16) en pas plus de cinq sections partielles séparées les unes des autres par la construction,
ou/et en dans lequel la formation de résistance à l'écoulement (38) réduit la section transversale pouvant être traversée du canal de gaz respiratoire aval (16) le long de la deuxième voie de canal (34) sur une longueur qui n'est pas inférieure à 5 mm.

2. Agencement de soupape expiratoire (10) selon la revendication 1, **caractérisé en ce que** la formation de résistance à l'écoulement (38) est disposée au moins dans une section axiale (L) du canal de gaz respiratoire aval (16) s'étendant le long de la deuxième voie de canal (34) dans la direction circonférentielle autour de la deuxième voie de canal (34) imaginée traversant centralement le canal de gaz respiratoire aval (16) sur une zone d'au moins 270° en formant un interstice radial à distance radiale de la paroi de canal (30a) du canal de gaz respiratoire aval (16).

3. Agencement de soupape expiratoire (10) selon la revendication 2, **caractérisé en ce que** l'interstice radial formé dans la section axiale (L) entre la formation de résistance à l'écoulement (38) et la paroi de canal (30a) du canal de gaz respiratoire aval (16) est un canal annulaire (39) fermé dans la direction circonférentielle.

4. Agencement de soupape expiratoire (10) selon la revendication 2 ou 3,
**caractérisé en ce que** l'interstice radial, en particulier sous forme de canal annulaire (39), présente sur au moins une partie de la section axial (L), de préférence sur la section axiale (L) entière, une forme constante ou/et une surface de section transversale constante pouvant être traversée.

5. Agencement de soupape expiratoire (10) selon la revendication 2 ou 3,
**caractérisé en ce que** la section axiale (L) s'étend jusqu'à l'extrémité longitudinale aval (16a) du canal de gaz respiratoire aval (16).

6. Agencement de soupape expiratoire (10) selon l'une quelconque des revendications précédentes,
**caractérisé en ce que** la formation de résistance à l'écoulement (38) dépasse axialement le canal de gaz respiratoire aval (16) au-delà de son extrémité longitudinale aval (16a).

7. Agencement de soupape expiratoire (10) selon l'une quelconque des revendications précédentes,
**caractérisé en ce que** la formation de résistance à l'écoulement (38) présente une extrémité aval (38a) effilée.

8. Agencement de soupape expiratoire (10) selon l'une quelconque des revendications précédentes,
**caractérisé en ce que** la formation de résistance à l'écoulement (38) présente, dans une portion d'extension (L) commune avec la paroi de canal (30a) du canal de gaz respiratoire aval (16), le long de la deuxième voie de canal (34), un contour extérieur, de forme et de taille constantes le long de la portion d'extension (L), de préférence un contour extérieur cylindrique.

9. Agencement de soupape expiratoire (10) selon l'une des revendications précédentes,
**caractérisé en ce que** la formation de résistance à l'écoulement (38) dépasse axialement le canal de gaz respiratoire aval (16) au-delà de son extrémité longitudinale amont (30c).

10. Agencement de soupape expiratoire (10) selon la revendication 9,
**caractérisé en ce que** les première et deuxième voies de canal (20, 34) forment un angle, de préférence un angle droit, l'une avec l'autre, dans lequel la formation de résistance à l'écoulement (38) fait saillie dans le passage de gaz respiratoire aval (16) à partir d'un élément de canal (18) délimitant le passage de gaz respiratoire amont (14).

11. Agencement de soupape expiratoire (10) selon la revendication 10,
**caractérisé en ce que** la formation de résistance à l'écoulement (38) est formée en une seule pièce avec l'élément de canal (18) du canal de gaz respiratoire amont (14).

12. Agencement de soupape expiratoire (10) selon l'une quelconque des revendications précédentes,
**caractérisé en ce qu'**il comprend une chambre annulaire (28) située en aval de l'agencement de soupape (26), entourant le canal de gaz respiratoire amont (14) et à partir de laquelle s'étend le canal de gaz respiratoire aval (16), dans lequel l'agencement de soupape (16) est disposé entre le canal de gaz respiratoire amont (14) et la chambre annulaire (28).

13. Agencement de soupape expiratoire (10) selon la revendication 12,
**caractérisé en ce que** le corps de soupape (24) est un corps de membrane (24) recouvrant une extrémité longitudinale (18a) du canal de gaz respiratoire amont (14) et **en ce que** le siège de soupape (22) est formé à l'extrémité longitudinale (18a) du canal de gaz respiratoire amont (14).

14. Agencement de soupape expiratoire (10) selon la revendication 13,
**caractérisé en ce qu'**il comporte un actionneur de soupape (40) par lequel le corps de membrane (24) peut être sollicité par une force de fermeture dans une direction de fermeture vers le siège de soupape (22).

15. Dispositif de ventilation pour la ventilation artificielle de patients avec un dispositif de livraison de gaz respiratoire, à partir duquel une ligne d'inspiration mène à une interface de ventilation du patient, à partir de laquelle à son tour une ligne d'expiration mène à un puits de gaz respiratoire (U), comme par exemple l'atmosphère ambiante (U),
**caractérisé en ce qu'**un agencement de soupape expiratoire (10) selon l'une des revendications précédentes est prévu dans la ligne d'expiration, dans lequel le canal de gaz respiratoire amont est relié par une section de la ligne d'expiration à l'interface patient-ventilateur pour le transfert de gaz respiratoire expiré depuis l'interface patient-ventilateur.
